# EUROPEAN PATENT APPLICATION

(11) **EP 2 527 346 A1**
(43) Date of publication of application: **28.11.2012**
(21) Application number: 11734326.9
(22) Date of filing: 19.01.2011
(51) Int. Cl.: C07F 5/00, C09K 11/06, C08L 101/08, C08K 5/00, G01N 33/52

(54) **PHOTOLUMINESCENT NANOPARTICLE, PREPARATION, AND APPLICATION THEREOF**

(30) Priority: 22.01.2010 CN 201010100090
(71) Applicant: Peking University, Beijing 100871 (CN)
(72) Inventor: WANG, Yuan, Beijing 100871 (CN); SHAO, Guangsheng, Beijing 100871 (CN); HAN, Rongcheng, Beijing 100871 (CN); YANG, Wen, Beijing 100871 (CN); MA, Yan, Beijing 100871 (CN); XUE, Fumin, Beijing 100871 (CN)
(74) Representative: Murgitroyd, Ian George
(86) International application number: PCT/CN2011/000082
(87) International publication number: WO 2011/088744

(57) **Abstract**

Luminescent nanoparticles, preparation, and application thereof are disclosed. The luminescent nanoparticle consists of matrix, which is a macromolecular compound containing carboxyl group, and a rare-earth luminescent dye dispersed in the matrix. The preparation method of the luminescent nanoparticle comprises: dissolving the rare-earth complex luminescent dye and the macromolecular compound in organic solvent miscible with water, adding the solution into water, and forming the luminescent nanoparticle by coprecipitation-selfassembly process. The prepared luminescent nanoparticle has excellent long-wave excitational luminescent properties and good stability, and can be used in coupling the surface carboxyl group of a biomolecule. The biological probes based on such luminescent nanoparticles have wide application prospects on the aspects of high-sensitivity luminescent immunoassay, biological imaging and the like.

## Description

### Technical field

The present invention relates to photoluminescent nanoparticles and lanthanide complex luminescent dyes useful to preparae such photoluminescent nanoparticles, as well as preparation methods and applications thereof, particularly, to luminescent nanoparticles that are based on the lanthanide complex luminescent dyes and preparation methods and applications thereof.

### Background art

The lanthanide complex-based luminescent probes are of important application values in the fields of biological imaging, DNA detection, immunoassay and the like. The main advantages thereof lie in that the lanthanide complexes have features such as long luminescence lifetime, large Stokes shift, sharp line emission peaks and the like. These features enable the lanthanide complex-based luminescent probes to be useful in effectively filtering out background signal interferences through time-resolved analysis technique in the biochemical assay so as to realize a high-sensitivity detection, meanwhile, avoiding biosafety problems caused by traditional radioactive labeling procedures.

Since the f-f transition of lanthanide ions is limited by the electric dipole selection rules, the lanthanide ions themselves have a very weak light absorption ability, they need to be sensitized in order to obtain a higher photoluminescent efficiency, that is, to absorb light through ligands (or charge transfer states) and transfer energy to excitation states of lanthanide ions, allowing realization of highly efficient luminescence of the lanthanide ions. Many lanthanide complexes can be excited by ultraviolet light, obtaining a good luminescence intensity. However, the ultraviolet exciting light causes great damages to organisms and has a strong background signal, meanwhile, the ultraviolet light has a small penetration depth, making applications of the ultraviolet excitation luminescent probes be substantively restricted. Therefore, it becomes one of the important development directions to extend the excitation window of the lanthanide complexes to a long-wavelength region, develope and prepare a lanthanide complex-based luminescent probe capable of being efficiently excitated by visible-light or near-infrared light.

The lanthanide complexes that have excellent luminescent properties under the excitation of visible-light and near-infrared light are very limited. In recent years, we have designed and synthesized an europium complex, Eu(tta)₃dpbt (dpbt=2-(N,N-diethylanilin-4-yl)-4,6-bis(3,5-dimethylpyrazol-1-yl)-1,3,5-triazine, wherein, tta is an anion of thenoyltrifluoroacetonato) (Y. Wang, et al., Angew. Chem. Int. Ed. 2004, 43, 5010). And based on this, a series of novel lanthanide complexes have been designed and synthesized, each of which has excellent luminescent properties under the excitation of visible-light or near-infrared light (Y. Wang, et al., Adv. Funct. Mater. 2007, 17, 3663; Y. Wang, R. Hao, Y. Ma, Europium Complexes Having Luminescent Properties Under Excitation of Visible-light and Method for Synthesizing the Same, ZL 2007 1 0118978.8). If such lanthanide complexes are used, as luminescent probes, into bioassay, the bioassay will be endowed with such advantages as high sensitivity, high signal-to-noise ratio, large penetration depth, low damage and interference to organisms, low assay cost etc. However, most of the known lanthanide complexes having highly efficient luminescence under the excitation of visible-light and near-infrared light are hydrophobic molecules and have low stability in an aqueous solution, thereby, it is difficult to employ directly these complex molecules as bioluminescent probes in an aqueous solution.

One of the feasible solutions to solve such a problem is to embed lanthanide complexes into polymer resins or inorganic nanoparticles. P. Xi et al. (P. Xi, X. H. Gu, X. A. Huang, Y. X. He, Inorganic-Organic Core-Shell Type Lanthanide Polymer Materials and Their Preparation Methods, CN1966534) embeded the hydrophobic lanthanide complexes having luminescent properties under the excitation of ultraviolet light into polymer resins by using the emulsion polymerization and swelling-embedding method, preparing nanoparticles having luminescent properties under the excitation of ultraviolet light; Y. Chen and J. L. Yuan (Y. Chen, Luminescent Lanthanide Nanoparticles Based on Luminescent Energy Transfer Principle and Their Preparation Methods, CN 1304523C; J. L. Yuan, M. Q. Tan, Z. Q. Ye, G. L. Wang, A Functional Lanthanide Nanoparticle, Preparation and Application Thereof, CN1298807C) embeded hydrophilic lanthanide complexes into nanoparticles wherein silicon dioxide serves as a matrix by using microemulsion method or sol-gel method respectively, preparing lanthanide complex-based nanomaterials having luminescent properties under the excitation of ultraviolet light. Whereas, nanoparticles using common hydrophobic macromolecules as matrixs are prone to aggregate in an aqueous solution, moreover, they have large particle size and strong scattering signal. Since silicon dioxide matrix is hard to form a dense encapsulating layer, it often results in leakage of the luminescent dyes in the formed nanoparticles. Jingli Yuan et al., using three compounds containing a siloxane group (APS-BHHCT-Eu³⁺-dpbt, 3-aminopropyl (triethoxyl) silane, tetraethyl orthosilicate), synthesized luminescent nanoparticles that can be excited by visible-light to emit light in a reverse microemulsion through a hydrolytic co-condensation method. (Jing Wu, Jingli Yuan, Yafeng Guan et al., Journal of Materials Chemistry, 2009, 19, 1258-1264). Such nanoparticles have amine groups on their surfaces, which may be used in coupling with a biomolecule, and the Eu³⁺ luminescent complexes are encapsulated within silica-based matrix materials. However, such nanoparticles exhibited strong scattering in the visible-light region, and the Eu³⁺ complexes in the particles showed a very small absorption peak in the UV-vis absorption spectrum thereof, which overlaps over a very strong scattering band, indicating the properties thereof such as photoluminescence intensity etc. are not desirable. In addition, the surfaces of such nanoparticles are positively charged in a neutral and weak acidic condition due to the presence of amine group, which are more likely to result in non-specific adsorption in a bioassay.

The ideal lanthanide complex-based luminescent nanoprobes should have the following features: 1) good dispersibility and stability in an aqueous solution; 2) the lanthanide complexes or aggregates thereof in the nanoparticles have strong photoluminescent properties, especially under the excitation of visible-light and near-infrared light, and the nanoparticles have a strong luminescent intensity but a weak scattering to the excitation light and emitting light; 3) the nanoparticles have an active group on their surfaces that is available for coupling a biomolecule, and good biocompatibility as well as low non-specific adsorption ability.

The present inventors had used the reported synthesis methods for lanthanide complex-based luminescent nanoparticles (e.g., microemulsion polymerization method, alkoxy silane hydrolysis and embedment method etc.) to perform an nano-embedment of the designed and synthesized lanthanide complexes, such as Eu(tta)₃dpbt and the like that have excellent luminescent properties under the excitation of visible-light and near-infrared light, so as to synthesize novel lanthanide complex-based luminescent nanoparticles, but had not obtain a desirable result. It mainly due to the weak coordination between the ligand of such lanthanide complex having excellent luminescent properties under the excitation of visible-light and near-infrared light and the lanthanide ion; conventional embedment method for complexes are prone to result in dissociation of the ligand thereof or quenched luminescence, thereby losing the original optical properties.

### Summary of the invention

The purpose of the present invention is to provide a kind of luminescent nanoparticles, preparation method and application thereof.

The luminescent nanoparticles provided by the present invention comprise a matrix material and lanthanide complexes dispersed in the matrix material.

The aforementioned luminescent nanoparticles may consist only of the matrix material and the lanthanide complexes dispersed in the matrix material. Wherein, the matrix material is macromolecular compounds formed of a backbone which is a hydrocarbon chain, and pendant carboxyl groups and hydrophobic groups; the lanthanide complex luminescent dyes are lanthanide complexes that emit visible-light or near-infrared light under excitation of visible-light and/or near-infrared light and/or ultraviolet light.

In the matrix material, the hydrophobic groups are selected from, but not limited to, at least one of alkyl, phenyl, ester groups and ether groups; in the matrix material, the hydrophobic groups are selected from at least one of alkyl, phenyl, ester group and ether group; the matrix material is selected from at least one of methacrylic acid-methacrylate copolymer, styrene-methacrylic acid copolymer, acrylic acid-acrylate copolymer, styrene-maleic acid copolymer, styrene-maleic anhydride copolymer, styrene-alkyl maleate-maleic acid copolymer, styrene-isobutyl maleate-maleic acid copolymer, alkyl maleate-maleic acid copolymer, polymer of monobutyl maleate and methyl vinyl ether, and styrene-methacrylate-acrylic acid copolymer, preferably at least one of methacrylic acid-methacrylate copolymer, styrene-methacrylic acid copolymer, styrene-maleic acid copolymer, styrene-alkyl maleate-maleic acid copolymer, polymer of monobutyl maleate and methyl vinyl ether, and styrene-isobutyl maleate-maleic acid copolymer, more preferably at least one of methacrylic acid-methyl methacrylate copolymer, styrene-methacrylic acid copolymer, styrene-maleic acid copolymer and styrene-isobutyl maleate-maleic acid copolymer; the lanthanide complex is selected from at least one of the compounds represented by the general structural formula of formula IV, the general structural formula of formula V, and the general structural formula of formula VI;

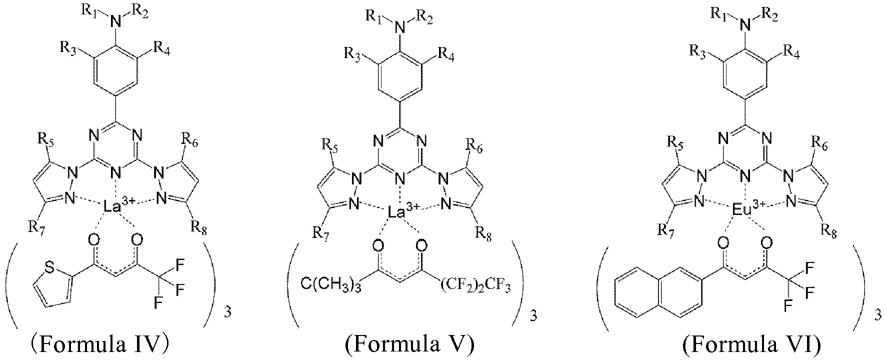

In the general structural formulas of formula IV, formula V and formula VI, La represents a europium, ytterbium or neodymium ion; R₁ and R₂ each is selected from any one of the alkyl groups with 1-4 carbon atoms; R₃, R₄, R₅, R₆, R₇ and R₈ each is selected from any one of methyl and H. The auxiliary ligand in formula IV is a thenoyltrifluoroacetonato anion (referred simply to as tta), the auxiliary ligand in formula V is a 6,6,7,7,8,8,8-heptafluoro-2,2-dimethyl octane-3,5-dione anion (referred simply to as fod), the auxiliary ligand in formula VI is a naphthoyltrifluoroacetone anion (referred simply to as nta).

The mass ratio of the matrix material to the lanthanide complex is 1∼10,000:1, particularly 1-2,000:1, 1-500:1, 1-100:1, 1-50:1, 1-5:1, 1-1.5:1, 5-2,000:1, 5-500:1, 5-100:1, 5-50:1, preferably 3-1000:1, more preferably 3-100:1; the macromolecular compound (i.e., the matrix material) has a number average molecular weight of 1,500-150,000, particularly 1,500-100,000, 1,900-100,000, 5,000-100,000, 5,000-65,000, 8,000-65,000, 8,000-10,000, 10,000-100,000, 10,000-65,000, 20,000-80,000, preferably 5,000-100,000, more preferably 10,000-70,000; in the macromolecular compound, the carboxyl group content is 0.01%∼40%, particularly 0.3-30%, 0.8-25%, 0.8-15%, 0.8-10%, 1-25%, 1-15%, 1-10%, 3-25%, 3-15%, 3-10%, 3-5%, 2-3%, 2-5%, 5-25%, preferably 0.05-20% of the total mass of the macromolecular compound; the luminescent nanoparticle has a particle size of 3-200 nanometers, particularly 3 nm ≤ particle size < 10nm, 5-190nm, 10-60nm, 12-180nm, 12-100nm, 12-145nm, 22-100nm, 30-180nm, 30-100nm, 35-145nm, 35-100nm, 40-180nm, 40-145nm, 40-100nm, 45-180nm, preferably 8-120 nm.

The method for preparing the aforementioned luminescent nanoparticle provided by the present invention comprises the following steps:
1) Dissolving the lanthanide complex and the matrix material in an organic solvent that is miscible with water to give an organic solution of the lanthanide complex and the matrix material;
2) Mixing the organic solution obtained from step 1) with water;
3) Removing the organic solvent in the mixture obtained from step 2), and redispersing the precipitate into water or buffer solutions after separation by centrifugation to give a sol of the luminescent nanoparticle dispersed into water or buffer solutions.

In step 1) of this method, the lanthanide complex has a concentration of 1×10⁻⁴∼10 g/L, preferably 0.01-3g/L; the matrix material has a concentration of 1×10⁻⁴∼100 g/L, preferably 0.01∼10g/L; the organic solvent that is miscible with water is selected from at least one of methanol, ethanol, acetone, acetonitrile, dimethylformamide and tetrahydrofuran;

In step 2), the volume ratio of water to the organic solution obtained from step 1) is 0.2-1000, preferably 0.5-100; particularly 0.2-800, 0.2-200, 0.2-100, 0.5-200, 0.6-200, 0.6-100, 0.6-50, 1-200, 1-100, 1-50, 3-200, 3-100, 3-5, 3-4, 4-200, 4-100, 4-5;

In step 3), in the step for removing the organic solvent in the mixture obtained from step 2), the temperature is 4∼100°C, preferably 4∼80 °C ; the buffer solution is selected from at least one of phosphate buffer solution, Tris-HCl buffer solution and carbonate buffer solution.

The aforementioned method for preparing luminescent nanoparticles is a coprecipitation-encapsulation process, that is, dissolving the lanthanide complex and the macromolecular compound into an organic solvent that is miscible with water, and adding the mixture into water to form nanoparticles of the matrix material that encapsulate the lanthanide complex luminescent dyes. By adjusting the ratio of the lanthanide complex and the macromolecular compound as well as the organic solvent, the parameters, such as the content of the lanthanide complex in the luminescent nanoparticle, particle size and the like can be conveniently regulated. While the content of the carboxyl group on the surface of the luminescent nanoparticle may be regulated by varying the composition of the macromolecular compound.

The present invention further provides a colloidal solution, which is formed by dispersing the foregoing luminescent nanoparticle provided by the present invention into water or a buffer solution.

In this colloidal solution, the buffer solution is selected from at least one of phosphate buffer solution, Tris-HCl buffer solution and carbonate buffer solution.

Uses of the foregoing luminescent nanoparticle and the colloidal solution formed by dispersing the foregoing luminescent nanoparticle into water or buffer solutions, provided by the present invention, in preparing luminescent biological nanoprobes or luminescent labels for biological imaging also belong to the protection scope of the present invention.

In order to prepare luminescent nanoparticles based on rare-earth complex luminescent dyes capable of being efficiently excited by visible-light and near-infrared light (e.g., the rare-earth complex luminescent dyes represented by formula IV, V, VI), which can be stably dispersed in an aqueous solution and contain carboxyl groups being prone to bond with biomolecules on the surfaces, the problem that the lanthanide complexes are vulnerable to damage during preparation and thereby lose the intrinsic luminescent properties thereof must be solved. This problem is mainly due to the weak coordination between the rare-earth ions and the ligands in the complexes with highly efficient rare-earth luminescence under the excitation of visible-light and near-infrared light (e.g., the lanthanide complex luminescent dyes represented by formula IV, V, VI), and many compounds containing amine group, carboxyl group or silicon hydroxyl group and the like (such as, acetic acid, n-butylamine, sodium silicate and the like) are prone to react with the complexes, making the complexes lose the long-wavelength sensitized luminescent properties. The present inventors found that carboxyl groups grafted to the hydrocarbon chain of the macromolecular compound with pendant hydrophobic groups has very weak interaction with the lanthanide complexes, such as the lanthanide complexes of formula IV, V, VI and the like having excellent luminescent properties under the excitation of visible-light and near-infrared light; once the mixture solution thereof is added into water, luminescent nanoparticles having excellent luminescent properties under visible-light excitation and two-photon excitation with near-infrared light (NIR) and good dispersion stability were created immediately (Yuan Wang et al., Chem. Eur. J. 2010, 16, 8647-8651). The occurrence of this unexpected phenomenon owes to stronger steric effect caused by the hydrophobic pendant groups and the hydrocarbon backbone of the macromolecular compounds, making it hard for the carboxyl groups grafted on such macromolecular compounds to interact with the aforementioned lanthanide complexes under experimental conditions in such a way that led to the decomposition of the coordination structure between the chromophores and the lanthanide ions, losing of the intrinsic optical properties thereof.

The macromolecular compound and the lanthanide complex are dissolved in an organic solvent that is miscible with water to give a solution; when the resultant solution is added into water under agitation, self-assembly occurs between the water-insoluble macromolecular compound and the lanthanide complex, wherein, the strong hydrophobic portion of the polymer and the hydrophobic lanthanide complex tend to get away from water, and part of the carboxyl groups in the polymer tend to expose to the surface and be ionized into carboxylate groups, making the surfaces of the nanoparticles be negatively charged. Since the size of the particles is small and the surface thereof is negatively charged, the luminescent nanoparticles provided by the present invention have very high dispersion stability in both water and a lot of buffer solutions. Because the lanthanide complex is dispersed in the medium constituted by the strong hydrophobic portion of the polymer, the luminescent nanoparticles provided by the present invention exhibit excellent luminescent properties under the long-wavelength light excitation and good photostability.

The aforementioned principles impart a wide range of options to the matrix material, the macromolecular compounds, of the present nanoparticles; therefore, simple replacement of the hydrophobic pendant group on the hydrocarbon chain of the macromolecular compound does not go beyond the protection scope of the present invention.

The present invention further provides a novel lanthanide complex having excellent photoluminescent properties which is served as a raw material for making the luminescent nanoparticle, and a method for preparing the same.

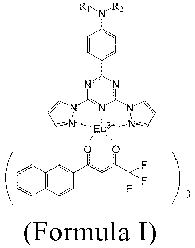

The lanthanide complex provided by the present invention, serving as a raw material for making the luminescent nanoparticle, has a general structural formula as represented by formula I. In the general structural formula of formula I, R₁ and R₂ each is selected from any one of the alkyl groups with 1-4 carbon atoms, preferably each is an ethyl.

The method for preparing the aforementioned europium ion complex provided by the present invention includes the following steps: reacting the compound represented by the general structural formula of formula II with that represented by the general structural formula of formula III to give said europium ion complex.

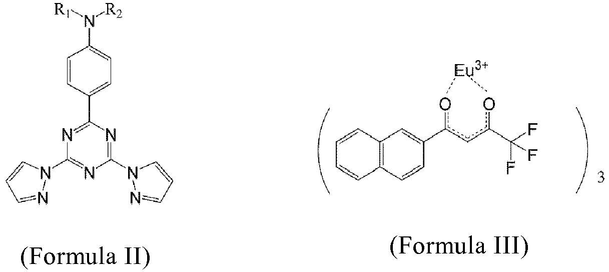

In the general structural formula of formula II, R₁ and R₂ each is selected from any one of the alkyl groups with 1-4 carbon atoms.

In this method, the molar ratio of the compound represented by the general structural formula of formula II to that represented by the general structural formula of formula III is 1:1; in the reaction, the temperature is -10∼100°C, preferably 20-30°C; the reaction is performed in the organic solvent, and the organic solvent is selected from at least one of tetrahydrofuran, ethyl ether, benzene, toluene, xylene, chloroform and dichloromethane.

### Brief description of the drawings

Figure 1 is a graph of particle size distribution of the Eu(tta)₃dpbt based luminescent nanoparticle prepared in Example 1 with methacrylic acid-methyl methacrylate copolymer as the matrix material (determined with the dynamic light scattering method, the nanoparticles have a particle size distribution ranging from 52 to 66 nm, an average particle size of 58 nm).
Figure 2 is a transmission electron microscope image of the luminescent nanoparticles prepared in Example 1.
Figure 3 is the ultraviolet-visible absorption spectrum of the luminescent nanoparticles prepared in Example 1.
Figure 4 is the excitation spectrum of the luminescent nanoparticles prepared in Example 1 (emission wavelength λₑₘ=614 nm).
Figure 5 is the emission spectrum of the luminescent nanoparticles prepared in Example 1 (excitation wavelength λₑₓ=412 nm).
Figure 6 is a graph showing the photostability of the luminescent nanoparticles prepared in Example 1 (excitation wavelength λₑₓ=412 nm, emission wavelength λₑₘ=614 nm).
Figure 7 is a graph showing the particle size distribution of the Eu(tta)₃dpbt complex-based luminescent nanoparticles prepared in Example 2 with styrene-methacrylic acid copolymer as the matrix material (determined with the dynamic light scattering method, the nanoparticles have a size range of 85∼115 nm, an average particle size of 100 nm).
Figure 8 is an ultraviolet-visible absorption spectrum of the luminescent nanoparticles prepared in Example 2.
Figure 9 is a luminescent excitation spectrum of the luminescent nanoparticles prepared in Example 2 (emission wavelength λₑₘ=614 nm).
Figure 10 is an ultraviolet-visible absorption spectrum of the Eu(nta)₃bpt based luminescent nanoparticles prepared in Example 3 with methacrylic acid-methyl methacrylate copolymer as the matrix material.
Figure 11 is an excitation spectrum of the luminescent nanoparticles prepared in Example 3 (emission wavelength λₑₘ=620 nm).
Figure 12 is an emission spectrum of the luminescent nanoparticles prepared in Example 3 (excitation wavelength λₑₓ=425 nm).
Figure 13 is a particle size distribution of the luminescent nanoparticles prepared in Example 3 (determined with the dynamic light scattering method, the nanoparticles have a size distribution ranging from 20 to 26 nm, an average particle size of 22 nm).
Figure 14 is an excitation spectrum of the luminescent nanoparticles prepared in Example 5 (emission wavelength λₑₘ=614 nm).
Figure 15 is an emission spectrum of the Nd(tta)₃dpbt based luminescent nanoparticles prepared in Example 11 with methacrylic acid-methyl methacrylate copolymer as the matrix material (excitation wavelength λₑₓ=412 nm).
Figure 16 is an emission spectrum of the Yb(tta)₃dpbt based luminescent nanoparticles prepared in Example 12 with methacrylic acid-methyl methacrylate copolymer as the matrix material (excitation wavelength λₑₓ=412 nm).
Figure 17 is an excitation spectrum of the Eu(tta)₃·3H₂O based luminescent nanoparticles prepared in Example 13 with acrylic acid-methyl methacrylate copolymer as the matrix material (emission wavelength λₑₘ= 614 nm).
Figure 18 is a graph showing that the luminescent intensity varies with the concentration of the human IgG in the detected sample in Example 16, wherein, the luminescent biological probe is the luminescent nanoparticle-labeled goat anti-human IgG.
Figure 19 is a graph showing that the luminescent intensity varies with the concentration of the human IgG in the detected sample in Example 16, wherein, the luminescent biological probe is a commercially available FITC-labeled goat anti-human IgG.
Figure 20 is the images of the Hela cervical carcinoma cells in Example 17, wherein, A is a bright field image, B is a luminescent image (excitation wavelength λₑₓ = 405 nm).
Figure 21 is an ultraviolet-visible absorption spectrum of a toluene solution of Eu(nta)₃bpt prepared in Example 18.
Figure 22 is an excitation spectrum of the toluene solution of Eu(nta)₃bpt prepared in Example 18 (emission wavelength λₑₘ=620 nm).
Figure 23 is an emission spectrum of the toluene solution of Eu(nta)₃bpt prepared in Example 18 (excitation wavelength λₑₓ=412 nm).

### Best mode for carrying out the invention

Several Examples are provided in order to describe the present invention in more detail, but the contents involved in the present invention are not limited by these Examples. All the methods used in the following Examples are conventional methods unless specifically indicated, and all reagents used in the Examples are commercially available.

### Example 1: Preparation of the Eu(tta)₃dpbt based luminescent nanoparticles with methacrylic acid-methyl methacrylate copolymer as the matrix material.

The methacrylic acid-methyl methacrylate copolymer (number average molecular weight, 50,000, mass fraction of the carboxyl group, 1.0 %) and Eu(tta)₃dpbt (having a structure as represented by formula VII) were dissolved in acetone so as to prepare an acetone solution, wherein the concentration of the acrylic acid-methyl methacrylate copolymer was 1 g/L and the concentration of Eu(tta)₃dpbt was 0.1 g/L. 20 mL of the aforementioned solution was dropped into 80 mL water under agitation, and a mixture was obtained after a continuous agitation for additional 10 min. The mixture was evaporated at 30°C to remove acetone, and isolated by centrifugation at a centrifugal speed of 25000 rpm (50000G). The resulting precipitate was re-dispersed into pure water to prepare a sol of the luminescent nanoparticles containing carboxyl groups on their surfaces.

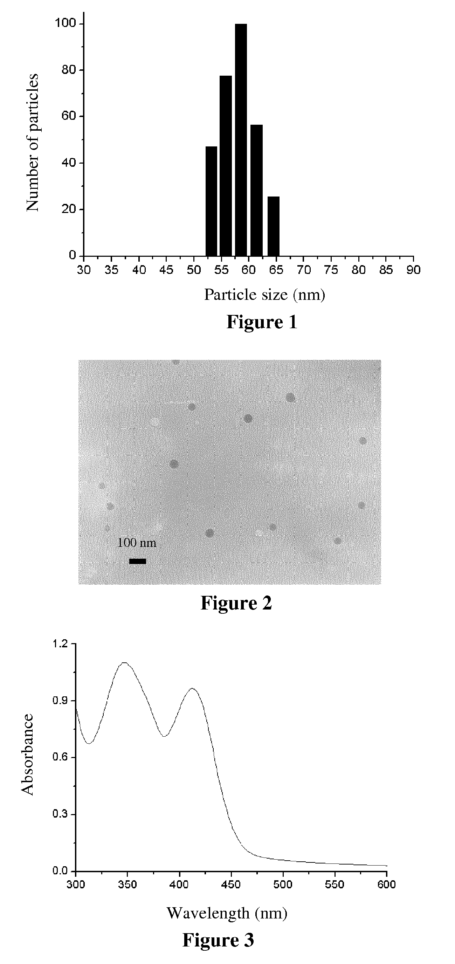

As shown in Figure 1, the dynamic light scattering measurement result indicates that the prepared luminescent nanoparticles have an average particle size of 58 nm, and a particle size distribution ranging from 52 to 66 nm. As shown in the transmission electron micrograph of the luminescent nanoparticle in Figure 2, the prepared luminescent nanoparticles are spherical in shape. The particle size distribution of the nanoparticles measured by transmission electron microscope (TEM) is consistent with the dynamic light scattering measurement result.

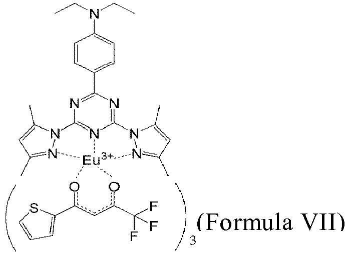

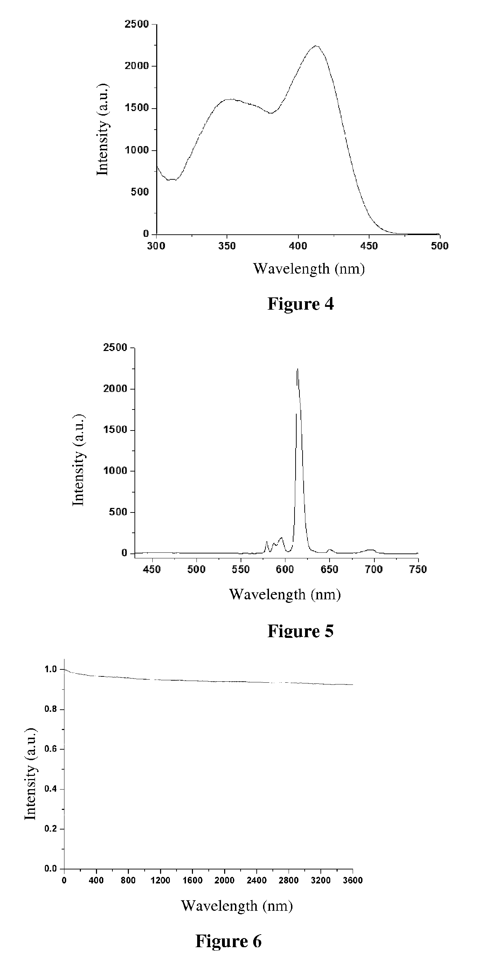

Figure 3 shows the ultraviolet-visible absorption spectrum of the sol of the prepared luminescent nanoparticle. As can be seen from this figure, the luminescent nanoparticles exhibit an absorption peak at 412 nm in the visible region, and the scattering of visible-light by the nanoparticles is quite weak. The luminescence spectrum measurement result indicates that the prepared luminescent nanoparticles have excellent luminescent properties under excitation of the visible-light, which has an excitation peak at 412 nm in the visible region with the tail extending up to 470 nm. The luminescent nanoparticles exhibit an excitation spectrum (λₑₘ=614 nm) as shown in Figure 4, and an emission spectrum (λₑₘ=412 nm) as shown in Figure 5. Using the n-propanol solution of 4-dicyanomethylene-2-methyl-6-p-dimethylaminostyrl-4H-pyran (referred simply to as DCM) as the reference (fluorescence quantum yield, *Φ* = 0.57), the quantum yield for the Eu³⁺ luminescence of the luminescent nanoparticles was measured to be 0.30 under the excitation at 410 nm (in all examples of the present invention, the quantum yield was calculated without deducting the scattering intensity where the light was absorbed). Under the excitation with the near-infrared laser possessing a wavelength of 830 nm, the aforementioned luminescent nanoparticles can emit bright red light, and the action cross section for the two-photon excitation thereof was measured to be 40 GM (1 GM = 10⁻⁵⁰ cm⁴ s photo⁻¹ molecule⁻¹) with the dye Rhodamine B as the reference. The aforementioned results demonstrate that the lanthanide complex Eu(tta)₃dpbt embedded in the prepared luminescent nanoparticle is structurally integral and maintains the excellent luminescent properties thereof. The prepared luminescent nanoparticles have good dispersion stability in an aqueous solution or phosphate buffer solution (PBS) with no precipitate being observed over a standing period of three months. As shown in Figure 6, the prepared luminescent nanoparticles exhibit excellent photostability, and the luminescence decay of the nanparticles is less than 10 % after light illumination for 1 hour at 412 nm. The Zeta potential of the prepared luminescent nanoparticles in water was measured to be -18 mV.

In this Example, Eu(tta)₃dpbt as represented by formula VII was prepared according to the method described in Angew. Chem. Int. Ed. 2004, 43, 5010-5013.

### Example 2: Preparation of the Eu(tta)₃dpbt based luminescent nanoparticles with styrene-methacrylic acid copolymer as the matrix.

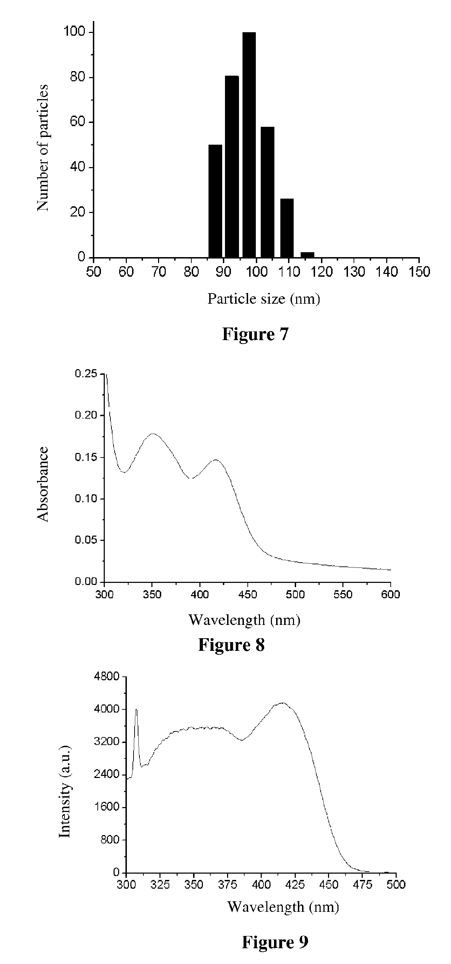

Eu(tta)₃dpbt based luminescent nanoparticles with styrene-methacrylic acid copolymer as the matrix were prepared according to the same method as that in Example 1 with the exceptions that the copolymer in Example 1 was replaced with styrene-methacrylic acid copolymer (number average molecular weight, 100,000, mass fraction of the styrene group: 40 %, mass fraction of the carboxyl group: 25 %), which had a concentration of 10 g/L, and the centrifugal speed was changed to 4000 rpm. As shown in Figure 7, the dynamic light scattering measurement result indicates that the luminescent nanoparticles as prepared have an average particle size of 100 nm, and a particle size distribution ranging from 85 to 115 nm. Figure 8 shows the ultraviolet-visible absorption spectrum of the sol of the luminescent nanoparticle as prepared, as can be seen from this figure, the luminescent nanoparticles exhibit an absorption peak at 415 nm in the visible region, and the scattering of visible-light by the nanoparticles is weak. The luminescent nanoparticles show an excitation spectrum (λₑₘ=614 nm) as shown in Figure 9 with an excitation peak at 415 nm and an excitation window in the visible region extending to more than 470 nm. The emission peak shape of the nanoparticles is similar to that of Figure 5. Under the illumination of an exciting light with a wavelength of 410 nm, the quantum yield for the Eu³⁺ luminescence of the luminescent nanoparticles was measured to be 0.31 with the n-propanol solution of DCM as the reference. The Zeta potential of the prepared luminescent nanoparticles in water was measured to be -28 mV.

### Example 3: Preparation of the Eu(nta)₃bpt based luminescent nanoparticles with methacrylic acid-methyl methacrylate copolymer as the matrix.

The methacrylic acid-methyl methacrylate copolymer (number average molecular weight: 5,000, mass fraction of the carboxyl group: 10 %) and Eu(nta)₃bpt (having a structure as represented by formula VIII, see Example 18 for the preparation method thereof) were dissolved in methanol to produce a methanol solution, wherein the concentration of the methacrylic acid-methyl methacrylate copolymer was 0.02 g/L, and the concentration of Eu(tta)₃bpt was 2×10⁻³ g/L. 25 mL of the aforementioned solution was dropped into 75 mL water under agitation, and a mixture was obtained after a continuous agitation for additional 10 min. Methanol was removed from this mixture by vacuum-rotary evaporation at 4°C. After isolation by centrifugation, the resulting precipitate was re-dispersed into pure water to prepare a sol of the luminescent nanoparticles containing carboxyl groups on their surfaces.

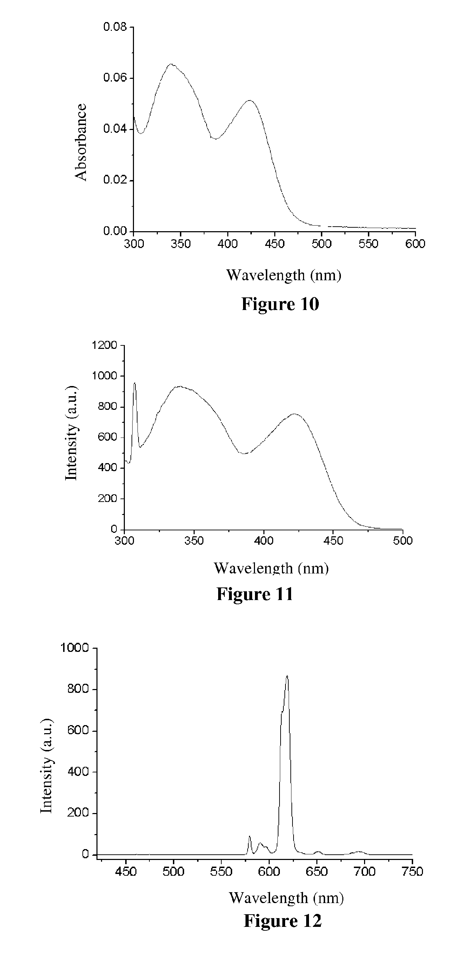

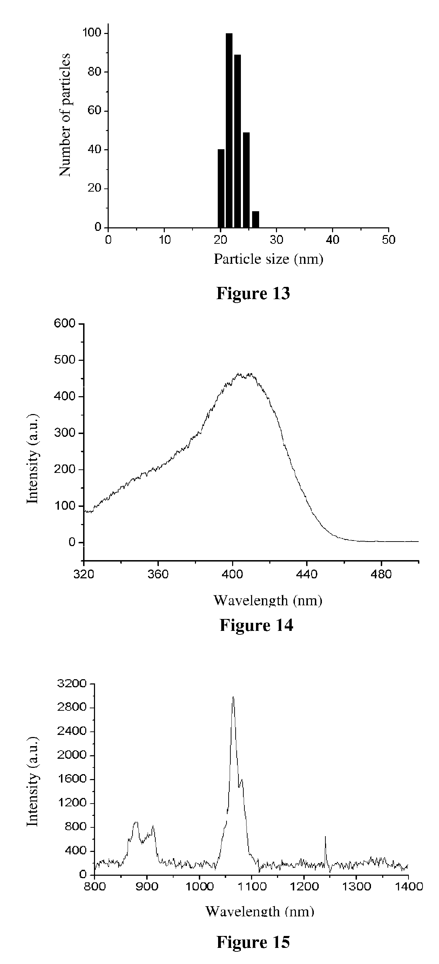

Figure 10 shows the ultraviolet-visible absorption spectrum of the sol of the luminescent nanoparticle as prepared, as can be seen from this figure, the luminescent nanoparticles show an absorption peak at 425 nm in the visible region, and the scattering of visible-light by the nanoparticles is quite weak. The luminescence spectrum measurement result indicates that the luminescent nanoparticle as prepared has excellent luminescent properties under excitation of visible-light, which has an excitation peak at 425 nm in visible region with the tail extending up to 470 nm. The luminescent nanoparticles exhibit an excitation spectrum (λₑₘ=614 nm) as shown in Figure 11, and an emission spectrum (λₑₘ=425 nm) as shown in Figure 12. As shown in Figure 13, the dynamic light scattering measurement results demonstrate that the luminescent nanoparticles as prepared have an average particle size of 22 nm, and a particle size distribution ranging from 20 to 26 nm. Under the optical excitation at 410 nm, the quantum yield for the Eu³⁺ luminescence of the luminescent nanoparticles was measured to be 0.30 with the propanol solution of DCM as the reference. The Zeta potential of the prepared luminescent nanoparticles in water was measured to be -21 mV, and the luminescence decay of the nanparticles is less than 10 % after light illumination at 415 nm for 0.5 hour.

### Example 4: Preparation of Eu(tta)₃dpbt based luminescent nanoparticles with styrene-maleic acid copolymer as the matrix material.

A styrene-maleic acid copolymer (number average molecular weight, 65,000, mass fraction of the carboxyl group: 20 %) and Eu(tta)₃dpbt were dissolved in acetone to prepare a acetone solution, wherein the concentration of the styrene-maleic acid copolymer was 10 g/L and the concentration of Eu(tta)₃dpbt was 1 g/L. 60 mL of the aforementioned solution and 40 mL water were mixed with the impinging stream-mixture method to give a mixture. Acetone in the mixture was removed by rotary evaporation at 50 °C, and the nanoparticles were isolated by centrifugation. The resulting precipitate was re-dispersed into pure water to prepare a hydrosol of the luminescent nanoparticles containing carboxyl groups on their surfaces.

The transmission electron microscope measurement results indicate that the luminescent nanoparticles as prepared have an average particle size of 7 nm, and a particle size distribution ranging from 3 to 10 nm. The luminescent nanoparticles exhibit an excitation spectrum (λₑₘ=614 nm) as shown in Figure 14. The luminescent nanoparticles as prepared show an excitation peak at 408 nm in visible region with the tail extending up to 460 nm, and their emission peak shape in the emission spectrum is similar to that as showed in Figure 5. Under the optical excitation at 410 nm, the quantum yield for the Eu³⁺ luminescence of the luminescent nanoparticles was measured to be 0.15 with the propanol solution of DCM as the reference. The luminescent nanoparticle as prepared has excellent dispersion stability in the aqueous solution or phosphate buffer solution (PBS). The Zeta potential measurement results indicate that the prepared luminescent nanoparticles dispersed in water have a zeta potential of -30 mV.

### Example 5: Preparation of Eu(tta)₃dpbt based luminescent nanoparticles with styrene-isobutyl maleate-maleic acid copolymer as the matrix material.

A styrene-isobutyl maleate-maleic acid copolymer (number average molecular weight, 10,000, mass fraction of the carboxyl group: 18 %) and Eu(tta)₃dpbt were dissolved in 50 mL acetone, to which 5 ml water was added to prepare a mixture, wherein the concentration of styrene-isobutyl maleate-maleic acid copolymer was 10 g/L and the concentration of Eu(tta)₃dpbt was 1 g/L. Acetone in the mixture was removed by rotary evaporation at 10 °C , and the nanoparticles were isolated by centrifugation. The resulting precipitate was re-dispersed into pure water to prepare a hydrosol of the luminescent nanoparticles containing carboxyl groups on their surfaces.

The transmission electron microscope measurement results indicate that the luminescent nanoparticles as prepared have an average particle size of 125 nm, and a particle size distribution range of 100∼140 nm. The excitation peak for the Eu³⁺ luminescence (λₑₘ=614 nm) of the nanoparticles has a similar shape to that as shown in Figure 14, and emission peak shapes of the emission spectrum are similar to those as shown in Figure 5. Under the optical excitation at 410 nm, the quantum yield for the Eu³⁺ luminescence of the luminescent nanoparticles was measured to be 0.13 with the propanol solution of DCM as the reference. The Zeta potential of the prepared luminescent nanoparticles in water was measured to be -26 mV.

### Example 6: Preparation of the Eu(fod)₃dpbt based luminescent nanoparticles with styrene-methacrylic acid copolymer as the matrix.

Eu(fod)₃dpbt based luminescent nanoparticles with styrene-methacrylic acid copolymer as the matrix was obtained according to the completely same method as that in Example 1 with the exception that the copolymer and the complex were replaced with styrene-methacrylic acid copolymer (number average molecular weight, 50,000, mass percentage of the carboxyl group, 2 %) and Eu(fod)₃dpbt (having a structure as represented by formula IX) respectively to be dissolved in the acetone to give a mixture, wherein the concentration of styrene-methacrylic acid copolymer was 25 g/L, and the concentration of Eu(fod)₃dpbt was 0.5 g/L.

The dynamic light scattering measurement result indicates that the luminescent nanoparticle as prepared has an average particle size of 145 nm. Under the optical excitation at 410 nm, the quantum yield for the Eu³⁺ luminescence of the luminescent nanoparticles was measured to be 0.25 with the propanol solution of DCM as the reference. The Zeta potential of the prepared luminescent nanoparticles in water was measured to be -15 mV.

In this Example, the Eu(fod)₃dpbt compound as represented by formula IX was prepared according to the following method: Eu(fod)₃, 0.05 mmol, was dissolved in 30 mL of tetrahydrofuran to prepare a solution D; the ligand compound dpbt, 0.05 mmol, was dissolved in 30 mL of tetrahydrofuran to prepare a solution E; solution D was added drop by drop into solution E. The mixture was agitated at 25 °C for 1 h, and the solvent was evaporated at reduced pressure. Eu(fod)₃dpbt was obtained as an orange-yellow solid, 72.7 mg. dpbt was prepared according to the method described in Angew. Chem. Int. Ed. 2004, 43, 5010-5013.

The molecular ion peak measured by mass spectrometry (MALDI-TOF MS) is M/Z =1454; elemental analysis (mass percentage): C, 43.66 % (43.78 %); H, 4.38 % (4.02 %); N, 7.43 % (7.71 %), wherein the values in the brackets are theoretical values; the nuclear magnetic resonance spectrum characterization proved that the product is the complex as represented by formula IX.

### Example 7: Preparation of the Eu(nta)₃bpt based luminescent nanoparticles with the mixture of methacrylic acid-methyl methacrylate copolymer and styrene-methacrylic acid copolymer as the matrix.

0.1 g of a methacrylic acid-methyl methacrylate copolymer (number average molecular weight, 50,000, mass percentage of the carboxyl group, 3%), 0.05 g of a styrene-methacrylic acid copolymer (number average molecular weight, 20,000, mass percentage of the carboxy group, 5%), and 0.1 g of Eu(nta)₃bpt (having a structure as represented by formula VIII) were dissolved in 10 ml acetone. The aforementioned solution was added into 90 ml water under agitation and ultrasound. Eu(nta)₃bpt based luminescent nanoparticles with the mixture of the above two copolymers as the matrix was prepared, according to the steps described in Example 1.

The dynamic light scattering measurement results indicate that the luminescent nanoparticle as prepared has an average particle size of 75 nm. The luminescence spectrum measurement results demonstrate that the luminescent excitation spectrum and emission spectrum thereof are similar with those of Figure 11 and Figure 12, respectively. The quantum yield for the Eu³⁺ luminescence of the luminescent nanoparticles was measured to be 0.30 with the propanol solution of DCM as the reference. The luminescent intensity of the luminescent nanoparticle is weakened by 10 % after light illumination at 415 nm for 1 hour.

### Example 8: Preparation of Eu(fod)₃dpbt based luminescent nanoparticles with methacrylic acid-methyl methacrylate copolymer as the matrix material.

Methacrylic acid-methyl methacrylate copolymer (number average molecular weight, 3,000, mass percentage of the carboxyl group, 15 %) and Eu(fod)₃dpbt (having a structure as represented by formula IX) were dissolved in acetonitrile to form a solution, wherein the concentration of methacrylic acid-methyl methacrylate copolymer was 1.0×10⁻³ g/L, and the concentration of Eu(fod)₃dpbt was 2.0×10⁻⁴ g/L. Under ultrasound conditions, 1 mL of the aforementioned solution was dropped into 100 mL water. Ultrasonic treatment was continued for an additional 10 min to give a mixture. The mixture was treated at 80°C to remove the acetonitrile, and isolated by centrifugation. The resulting precipitate was re-dispersed into phosphate buffer solution to prepare a sol of the luminescent nanoparticle containing carboxyl groups on their surfaces, wherein the luminescent nanoparticles were the Eu(fod)₃dpbt based luminescent nanoparticles with the methacrylic acid-methyl methacrylate copolymer as the matrix material.

The dynamic light scattering measurement results indicate that the luminescent nanoparticles as prepared have an average particle size of 12 nm. Under the optical excitation at 410 nm, the quantum yield for the Eu³⁺ luminescence of the luminescent nanoparticles was measured to be 0.27 with the propanol solution of DCM as the reference. The Zeta potential of the prepared luminescent nanoparticles in water was measured to be -20 mV.

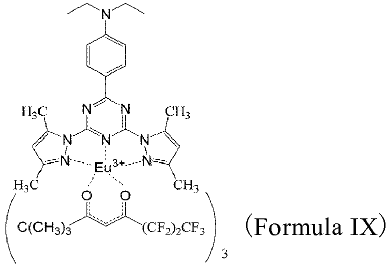

### Example 9: Preparation of Eu(nta)₃bpt based luminescent nanoparticles with styrene-methacrylic acid block copolymer as the matrix material.

Styrene-methacrylic acid block copolymer (number average molecular weight, 50,000, mass percentage of the carboxyl group, 0.5 %) and the Eu(nta)₃bpt (having a structure as represented by formula VIII) as prepared in Example 3 were dissolved in tetrahydrofuran to prepare a solution, wherein the concentration of styrene-methacrylic acid block copolymer was 50 g/L, and the concentration of Eu(nta)₃bpt was 1g/L. Under ultrasound irradiation, 10 mL of the aforementioned solution was dropped into 50 mL water. Ultrasonic treatment was continued for an additional 10 min to give a mixture. Tetrahydrofuran in the mixture was removed by rotary evaporation at 15°C, and the nanoparticles were isolated by centrifugation. The resulting precipitates were re-dispersed into a Tris-HCl (pH=8) buffer solution to prepare a sol of the luminescent nanoparticle containing carboxyl groups on their surfaces, wherein the luminescent nanoparticles are the Eu(nta)₃bpt based luminescent nanoparticles with the styrene-methacrylic acid block copolymer as the matrix material.

The dynamic light scattering measurement result indicates that the luminescent nanoparticles as prepared have an average particle size of 180 nm. The excitation peak for the Eu³⁺ luminescence (λₑₘ=614 nm) of the nanoparticles has a similar shape to that as shown in Figure 11, and emission peak shapes of the emission spectrum are similar to those as shown in Figure 12. Under the optical excitation at 410 nm, the quantum yield for the Eu³⁺ luminescence of the luminescent nanoparticles was measured to be 0.30 with the propanol solution of DCM as the reference.

### Example 10: Preparation of Eu(tta)₃dpbt based luminescent nanoparticles with methacrylic acid -methyl methacrylate copolymer as the matrix material.

Methacrylic acid-methyl methacrylate copolymer (number average molecular weight: 10,000, mass fraction of the carboxyl group: 5 %) and Eu(tta)₃dpbt were dissolved in dimethylformamide to prepare a solution, wherein the concentration of methacrylic acid-methacrylate copolymer was 0.2 g/L, and the concentration of Eu(tta)₃dpbt was 0.02 g/L. Under magnetic stirring, 10 mL of the aforementioned solution was dropped into 10 mL of water. Stirring was continued for an additional 15 min to give a mixture. Heating under reflux was performed on the mixture at 100 °C for 40 min, followed by separation through centrifugation. The resulting precipitate was re-dispersed into deionized water to prepare a sol of the luminescent nanoparticles containing carboxyl groups on the surfaces, wherein the luminescent nanoparticles are the Eu(tta)₃dpbt based luminescent nanoparticles with the methacrylic acid-methyl methacrylate copolymer as the matrix material.

The average particle size of the luminescent nanoparticles was measured to be 40 nm by the dynamic light scattering method. The luminescence excitation spectrum (λₑₘ=614 nm) shows an excitation peak at 412 nm in the visible region. Under the optical excitation at 410 nm, the quantum yield for the Eu³⁺ luminescence of the luminescent nanoparticles was measured to be 0.31 with the propanol solution of DCM as the reference.

### Example 11: Preparation of Nd(tta)₃dpbt based luminescent nanoparticles with methacrylic acid-methyl methacrylate copolymer as the matrix material.

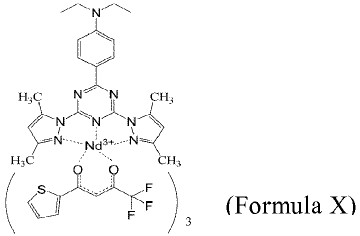

Nd(tta)₃dpbt based luminescent nanoparticles with methacrylic acid-methyl methacrylate copolymer as the matrix material was obtained using the completely same preparation steps as those in Example 10 with the exceptions that Eu(tta)₃dpbt in Example 10 was replaced Nd(tta)₃dpbt, of which the concentration was 1×10⁻⁴ g/L.

The average particle size of the prepared luminescent nanoparticles was measured to be 45 nm by the dynamic light scattering method. The luminescence emission spectrum of the prepared nanoparticles as shown in Figure 15 exhibits a main emission peak centered at 1064 nm in the near-infrared region. The Zeta potential of the prepared luminescent nanoparticles in water was measured to be -24 mV.

In this Example, the compound Nd(tta)₃dpbt (having a structural formula as represented by formula X) was prepared according to the following method:
Under stirring, 1.0 mmol of Nd(NO₃)₃·6H₂O was dissolved in 15 ml of ethanol to give a solution, which was added drop by drop into 15 ml of ethanol solution containing 3.0 mmol of thenoyltrifluoroacetone. The mixture was stirred at room temperature, to which an appropriate amount of ammonia liquor was added simultaneously, and the pH was adjusted to 7.0. The resulting mixture was heated at 80-85 °C to react for 3 h, which was then cooled to room temperature. Ethanol in the mixture was removed by vacuum-rotary evaporation to give a light blue solid, which was washed repeatedly with water prior to the addition of 10 ml of benzene to dissolve the solid, and then the mixture was dried with anhydrous calcium chloride. The calcium chloride was filtered out and solvent was removed by vacuum-rotary evaporation to give a blue oily substance, to which 10 ml petroleum ether was added and the mixture was heated to get a solution. After filtering out insoluble matters, petroleum ether in the mixture was removed by evaporation, and the obtained solid was dried in a vacuum oven to give a blue pulverulent product, Nd(tta)₃·2H₂O.

0.05 mmol of Nd(tta)₃·2H₂O was dissolved in 30 mL of tetrahydrofuran to prepare a solution B; 0.05 mmol of dpbt was dissolved in 30 mL of tetrahydrofuran to prepare a solution C; solution B was added drop by drop into the solution C. The mixture was stirred at 25°C for 1h, and the solvent was evaporated at reduced pressure to give an orange-yellow solid, Nd(tta)₃dpbt. Wherein, the dpbt ligand was prepared according to the method described in Angew. Chem. Int. Ed. 2004, 43, 5010-5013.

The mass spectrometry (ESI-MS) characterization result: [M+ Na]⁺ = 1247.3; elemental analysis (mass percentage): C, 46.18 %(46.11 %); H, 3.62 %(3.29 %); N, 8.99 % (9.15 %), wherein values in the brackets are theoretical values; nuclear magnetic resonance (NMR) spectrum characterization proves that the product is the Nd(tta)₃dpbt complex.

### Example 12: Preparation of Yb(tta)₃dpbt based luminescent nanoparticles with methacrylic acid-methyl methacrylate copolymer as the matrix material.

Yb(tta)₃dpbt based luminescent nanoparticles with methacrylic acid-methyl methacrylate copolymer as the matrix material was obtained by conducting the same operable steps as those in Example 10 except that Eu(tta)₃dpbt in Example 10 was replaced with Yb(tta)₃dpbt.

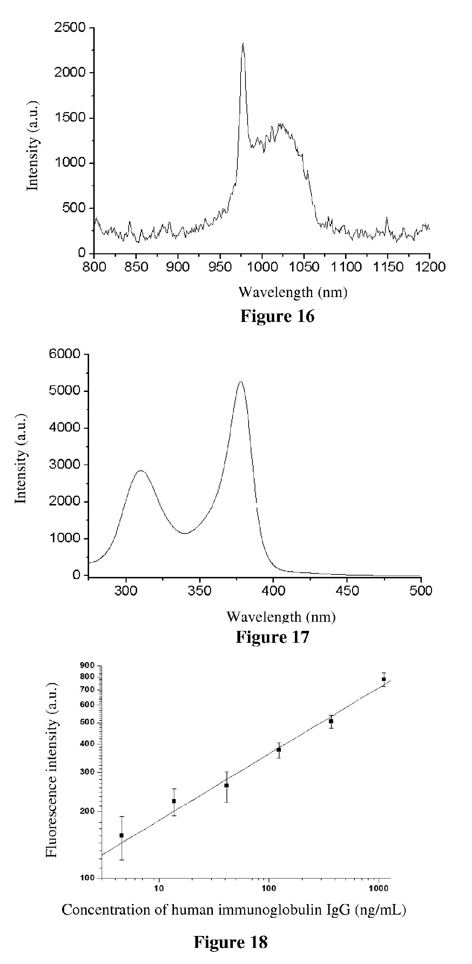

The average particle size of the prepared luminescent nanoparticles was measured to be 35 nm by the dynamic light scattering method. The excitation peak in the visible region for the Yb luminescence of the prepared nanoparticles locates at 412 nm, and the luminescence emission spectrum is shown in Figure 16 which exhibits two main emission peaks at 977 nm and 1024 nm, respectively, in the near-infrared region. The Zeta potential of the prepared luminescent nanoparticles in water was measured to be -18 mV.

In this Example, the complex, Yb(tta)₃dpbt, was synthesized and obtained according to the steps described in Example 11 with Nd(NO₃)₃·6H₂O being replaced with Yb(NO₃)₃·6H₂O, which has a structure as represented by formula XI.

The mass spectrometry (ESI-MS) characterization measurement result: [M+ Na]⁺ = 1276.1; elemental analysis (mass percentage): C, 44.31 % (45.05 %); H, 3.38 % (3.22 %); N, 9.47 %(8.94 %), wherein values in the brackets are theoretical values; The NMR spectrum of the product produced according to the aforementioned method proves that it is Yb(tta)₃dpbt.

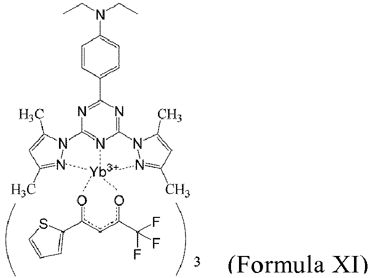

### Example 13: Preparation of Eu(tta)₃·3H₂O based luminescent nanoparticles with acrylic acid-methyl methacrylate copolymer as the matrix material.

Acrylic acid-methyl methacrylate copolymer (number average molecular weight: 15,000, mass percentage of the carboxyl group, 5 %) and Eu(tta)₃·3H₂O (purchased from ACROS Company) were dissolved in acetone to prepare an acetone solution, wherein the concentration of acrylic acid-acrylate copolymer was 5 g/L, and the concentration of Eu(tta)₃·3H₂O was 5 g/L. Under magnetic stirring condition, 500 µL of the aforementioned solution was injected into 100 mL water with a microinjector, followed by a continue agitation for additional 10 min. The sol was treated at 40 °C to remove acetone and isolated by centrifugation, and then, the resulting precipitate was re-dispersed into deionized water to prepare a sol of the luminescent nanoparticles containing carboxyl groups on the surfaces, wherein the luminescent nanoparticles are the Eu(tta)₃·3H₂O based luminescent nanoparticles with acrylic acid-methyl methacrylate copolymer as the matrix material.

The dynamic light scattering measurement results indicate that the luminescent nanoparticles as prepared have an average particle size of 30 nm. The luminescence excitation spectrum (λₑₘ=614 nm) of the prepared nanoparticles in the sol shows an excitation peak at 377 nm. Under the optical excitation at 377 nm, the quantum yield for the Eu³⁺ luminescence of the luminescent nanoparticles was measured to be 0.35. Figure 17 shows the excitation spectrum of the luminescent nanoparticles prepared in this experiment, exhibiting that such luminescent nanoparticles have good luminescent properties under the excitation of ultraviolet light.

### Example 14: Preparation of Eu(tta)₃bpt based luminescent nanoparticles with the copolymer of monobutyl maleate and methyl vinyl ether as the matrix material.

The copolymer of monobutyl maleate and methyl vinyl ether (number average molecular weight: 8,000, mass fraction of the carboxyl group: 0.5 %) and Eu(tta)₃bpt were dissolved in acetonitrile to prepare a solution, wherein the concentration of the copolymer of monobutyl maleate and methyl vinyl ether was 5 g/L, and the concentration of Eu(tta)₃bpt was 0.01 g/L. Under ultrasound irradiation, 1 mL of the aforementioned solution was injected into 100 mL water with an injector, followed by continuous ultrasonic treatment for additional 10 min. The resulting mixture was evaporated at 80 °C in a rotary vacuum evaporator to remove acetonitrile and treated by centrifugation. The resulting precipitate was re-dispersed into phosphate buffer solution to prepare a sol of the luminescent nanoparticles containing carboxyl groups on their surfaces, wherein the luminescent nanoparticles are the Eu(tta)₃bpt based luminescent nanoparticles with the copolymer of monobutyl maleate and methyl vinyl ether as the matrix material.

The dynamic light scattering measurement result indicates that the luminescent nanoparticle as prepared has an average particle size of 80 nm. The luminescence spectrum measurement result demonstrates that the nanoparticles as prepared above have good luminescent properties under the excitation of visible-light, with an excitation peak at 425 nm in the visible region. Under the optical excitation at 410 nm, the quantum yield for the Eu³⁺ luminescence of the luminescent nanoparticles was measured to be 0.24 with the propanol solution of DCM as the reference.

### Example 15: Preparation of Yb(tta)₃dpbt based luminescent nanoparticles with styrene-maleic anhydride copolymer as the matrix material.

The preparation conditions were same as those in Example 4 except that the styrene-maleic acid copolymer and the complex Eu(tta)₃dpbt in Example 4 were replaced with styrene-maleic anhydride copolymer (number average molecular weight, 1900, mass fraction of the carboxyl group: 12 %) and Yb(tta)₃dpbt, respectively.

The transmission electron microscope measurement result indicates that the luminescent nanoparticles as prepared have an average particle size of 26 nm, and a particle size distribution ranging from 16 to 40 nm. The luminescent nanoparticles have a peak shape of the excitation spectrum (λₑₘ=614 nm) similar to that as shown in Figure 14, and an emission peak shape of the emission spectrum similar to that as shown in Figure 16. The Zeta potential of the prepared luminescent nanoparticles dispersed in water was measured to be -22 mV.

### Example 16: Synthesis of a luminescent biological nanoprobe (the luminescent nanoparticles labeled goat anti-human IgG) and confirmation of the effect thereof.

The luminescent nanoparticles (LNP) as prepared in Example 2 was used as a label particles to label goat anti-human IgG, and the solid phase sandwich luminescent immunoassay was used for quantitative detection of human IgG. The specific methods are described as follows:
10 ml of the sol of the luminescent nanoparticles as prepared in Example 2 were taken, washed by centrifugation, and then dispersed in 10 ml of PBS(10 mM), to which 0.2 ml of a goat anti-human IgG solution with a concentration of 10 mg/ml, 0.2 ml of a N-(3-dimethylaminopropyl)-N'-ethylcarbodiimide (EDC) solution with a concentration of 100 mg/ml, and 0.1 ml of a N-hydroxysuccinimide (NHS) solution with a concentration of 100 mg/ml were added, followed by a incubation in a shaker at 25 °C for 4.0 h. Washing was conducted three times by centrifugation to remove excessive goat anti-human IgG, EDC and NHS, thereafter, the product was dispersed into 10 mM PBS containing 1 mg/ml bovine serum albumin (BSA), thereby obtaining the luminescent probe anti-IgG@LNP for detecting human IgG, which was stored at 4 °C for use.

Goat anti-human IgG was dispersed into 50 mM sodium carbonate-sodium bicarbonate buffer solution, pH=9.16, and its concentration was made to 5µg/mL. This solution was added into an ELISA plate, 100 *µ*L for each well, which was then maintained at 4°C overnight. The goat anti-human IgG solution was poured out, the ELISA plate was then washed three times with a 10 mM PBS-Tween-20 buffer solution (pH = 7.4, containing 0.5% Tween-20). After that, a 10 mM PBS-Tween-20 buffer solution of BSA (1.0%) was added into the above ELISA plate, 100 *µ*L for each well, which was placed into a thermostatic homogenizer maintaining at 25 °C for 1.0 h. The BSA solution was poured out, the ELISA plate was then washed three times with a 10mM PBS-Tween-20 buffer solution. Different concentration of human IgG standard solutions were added into different wells of the plate, which were incubated at 37 °C for 1.0 h. The human IgG solution was poured out, the ELISA plate was then washed three times with a 10 mM PBS-Tween-20 buffer solution. The sample wells were added with the luminescent probe anti IgG@LNP as prepared, and meanwhile, the control well was added with the goat anti-human IgG labeled with the commercially available FITC label. Incubation was conducted at 37°C for 1.0 h. Finally, excessive solutions were poured out, the ELISA plate was then washed five times with a 10 mM PBS-Tween-20 buffer solution.

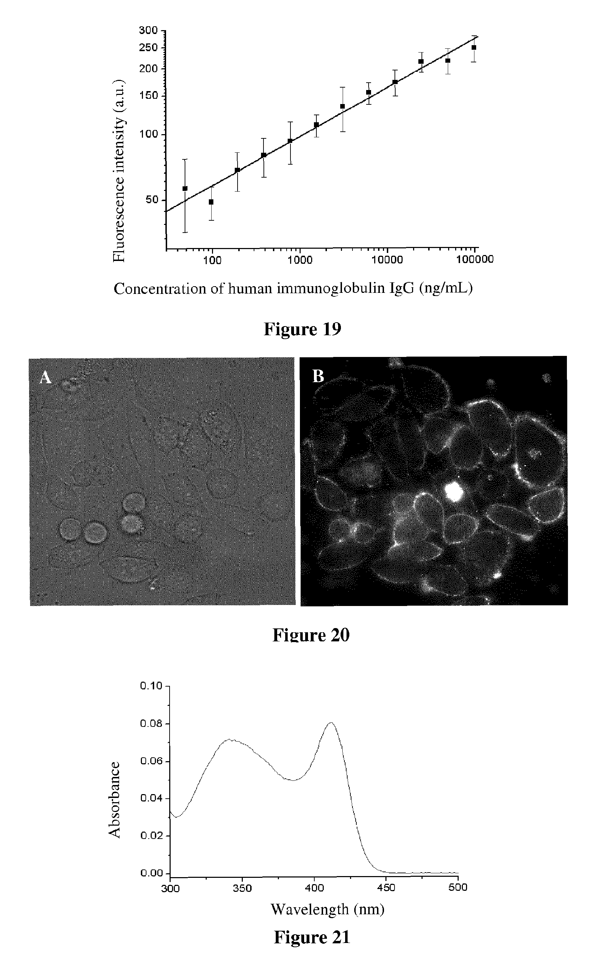

Multifunctional ELISA instrument was used to determine the luminescent intensity for each well of the ELISA plate. The sample well were measured with a time-resolved mode, with an excitation wavelength of 410 nm and a detection wavelength of 614 nm, the experimental results are shown in Figure 18; the control well was measured with an excitation wavelength of 485 nm and a detection wavelength of 525 nm, the experimental results are shown in Figure 19. The experimental results show that the synthesized luminescent nanoprobe anti-IgG@LNP has a detection limit of the luminescent immunoassay of 10 ng/mL for human IgG, and the commercially available FITC labeled goat anti-human IgG luminescent probe has a detection limit of the luminescent immunoassay of 500 ng/mL for human IgG.

Example 17: Use of the luminescent biological nanoprobe in imaging for cervical carcinoma cell.

Folic acid was coupled on the surface of the luminescent nanoparticles as prepared in Example 3. The biological probe as formed was employed to act with the cervical carcinoma cell, Hela. The luminescence imaging for Hela cell was conducted under the emission microscopy. The specific methods are described as follows:
10 ml of the sol of the luminescent nanoparticles as prepared in Example 3 was taken and isolated by centrifugation, and the precipitate was dispersed into 10 ml of PBS buffer t solution (10 mM), to which 0.2 ml of DEC solution with a concentration of 100 mg/ml, and 0.1 ml of NHS solution with a concentration of 100 mg/ml were added. An activating reaction was performed at 25 °C for 1.0 h, the reactant was isolated by centrifugation, and the precipitate was washed with 10 ml of PBS (10 mM) solution and re-dispersed into 10 ml of PBS (10 mM), to which, 0.1 ml folic acid solution with a concentration of 10 mg/ml was added to react for 4.0 h. The obtained reaction mixture was centrifugated, and the precipitate was washed with 10 ml of PBS (10 mM) solution, the excessive folic acid was removed and then the product was dispersed into a 10 mM PBS solution containing 1 mg/ml BSA, thereby obtaining a luminescent nanoprobe FA@LNP with the surface being modified by folic acid, which was stored at 4°C for use.

Hela cell in logarithmic growth phase was taken and digested with 0.25% trypsin, and then, inoculated into a confocal petri dish at a density of 3×10⁴ cell/cm². The petri dish was added with 0.5 ml Dulbecco's modified eagle's medium (DMEM medium), cultivated in an incubator containing 5 % CO₂ at 37 °C for 24 h. Thereafter, the petri dish was taken out, rinsed with 10 mM PBS solution to remove the medium, luminescent probe FA@LNP was diluted 10-fold with the DMEM medium and added into the petri dish, and the system incubated at ambient temperature for 2 h. Then, the petri dish was rinsed three times with 10 mM PBS to remove the medium and excessive luminescent nanoparticles. Luminescence microscopy was used to conduct imaging for Hela cell (Figure 20). The bright field imaging picture (Figure 20A) indicates that the cells are in a good state and the probe does not result in cell death, suggesting that the luminescent probe FA@LNP has a low toxicity. The luminescence image is shown in Figure 20B, and the excitation wavelength is 405 nm. Figure 20B shows that the luminescent probe FA@LNP is specifically attached to the surface of Hela cell, and no obvious aggregation of FA@LNP occurred.

### Example 18: Preparation of the Eu(nta)₃bpt compound as represented by formula VIII.

Under the protection of argon, 100 mmol of diethylaniline and 50 mmol of tripolychlorazine were stirred at 80-90°C for 8-9 h. The resulting solution after reaction was cooled to room temperature, to which 30 ml benzene was added. The insoluble matters were filtered out. Then, the resulting solution was evaporated at reduced pressure to remove the solvent, and the residue was isolated by a silica gel column with petroleum ether as the eluent, thereby obtaining the product, 2-(N,N-diethylanilin-4-yl)-4,6-dichloro-1,3,5-triazine, 5g.

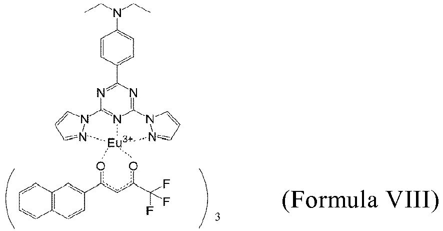

Under the protection of argon, 1.25 mmol of freshly cut potassium metal was added into an anhydrous tetrahydrofuran, to which 1.75 mmol of pyrazole was added; heating under reflux was performed for 3h to create a pyrazole anion; the reacting mixture was placed in an ice bath, to which 0.5 mmol *2-(N,N-*diethylanilin-4-yl)-4,6-dichloro-1,3,5-triazine was added, stirred at room temperature for 1h, and then, subjected to reflux reaction in an oil bath at 80-85 °C for 8-9 h. The reacting solution was evaporated at reduced pressure to remove the solvent after cooling down, and the residue was isolated by a silica gel column with the mixture solution of dichloromethane and ethyl acetate as the eluent. The resulting crude product was recrystallized with the mixture solution of petroleum ether and dichloromethane to give a yellow needle-like crystal product, 2-(*N,N*-diethylanilin-4-yl)-4,6-bis(pyrazol-1-yl)-1,3,5-triazine (referred simply to as bpt), 72 mg.

3 mmol 4,4,4-trifluoro-1-(2-naphthol)-1,3-butanedione (nta) was dissolved in 15 ml of anhydrous ethanol, to which 3 ml of aqueous solution containing 1 mmol sodium hydroxide was added under agitation, followed by the sequential addition of 5 ml aqueous solution containing 1 mmol of europium chloride hexahydrate and about 100 ml water. The resulting reaction solution was heated to 60 °C, stirred to react for 4 h, and then, cooled to room temperature, and filtered. The resulting solid was dried in a vacuum oven at room temperature prior to dissolving in 50 ml anhydrous ethanol. Then, heat filtration was performed and, 100 ml water was added to the filtrate. The resulting precipitate was frozen, and the solid obtained through filtration was dried in a vacuum oven, the obtained solid was washed with an appropriate amount of n-hexane, collected, and dried in vacuum at 50°C to give a product, Eu(nta)₃·H₂O, 0.85 g.

53.7 mg of the aforementioned complex and 20 mg of bpt were dissolved into 10 ml of tetrahydrofuran respectively, and the former was slowly dropped into the later, and reaction was conducted at room temperature for 2 h. The reaction mixture was evaporated to dryness in a rotary vacuum evaporator, a small amount of anhydrous ethyl ether was added to the solid, and then an appropriate amount of n-hexane was added. The solid was collected after precipitation, and dried in a vacuum oven at 70°C to give a product, Eu(nta)₃dpbt, 58 mg.

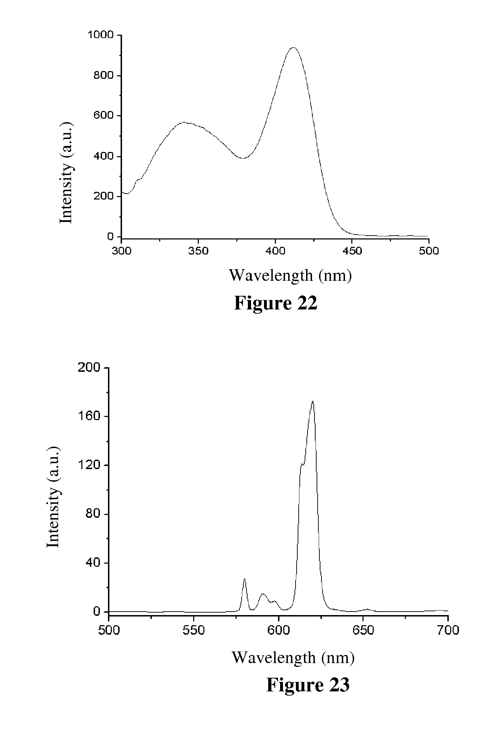

The molecular ion peak measured by the mass spectrometry (EI MS) is M/Z = 1182(M-naphen)⁺; elemental analysis (mass percentage) results: C, 55.83 % (56.01 %); H, 3.53 % (3.39 %); N, 8.56 % (8.57 %), wherein values in the brackets are theoretical values; NMR measurement prove that the product is the complex as represented by formula VIII. Figure 21 shows the ultraviolet-visible absorption spectrum of a toluene solution of Eu(nta)₃bpt as prepared. As can be seen from this figure, in the visible region there is an absorption peak at 412 nm, indicating that there is a red-shift up to 10 nm relative to the absorption peak of Eu(tta)₃dpbt. The excitation spectrum (λₑₘ=620 nm) of the toluene solution of the Eu(nta)₃bpt compound is shown in Figure 22. Eu(nta)₃bpt has excellent luminescent properties under excitation of the visible-light, with an excitation peak at 412 nm in the visible region and the tail extending up to 460 nm. The emission spectrum (λₑₘ=410 nm) is shown in Figure 23. In the Eu(nta)₃bpt compound, the emission peak of ⁵Do → ⁷F₂ transition of the Eu³⁺ is different from that of the Eu(tta)₃dpbt, demonstrating that the coordination environment of the Eu³⁺ ion is greatly altered due to the change of ligand.

Under the optical excitation at 410 nm, the quantum yield for the Eu³⁺ luminescence of the luminescent nanoparticles was measured to be 0.52 at 20□ with the propanol solution of DCM as the reference, which is increased by 30% relative to that of Eu(tta)₃dpbt.

### Industrial application

The luminescent nanoparticles provided by the present invention have the following advantages:
1. Excellent luminescent properties. The present luminescent nanoparticles possess advantages based on the lanthanide complexes, such as narrow emission peak, large Stocks shift, and long luminescence lifetime. The present luminescent nanoparticles exhibit weak scattering of exciting light and emitting light in both aqueous solution and buffer solution, and a high luminescent brightness. When the lanthanide complexes having luminescent properties under the excitation of visible and near-infrared light are used as luminescent dyes, the nanoparticles exhibit quite high quantum yields for lanthanide luminesce under the excitation of visible-light and near-infrared light, for example, the Eu(tta)₃dpbt, Eu(fod)₃dpbt or Eu(nta)₃bpt based luminescent nanoparticles as prepared in Examples 1 to 3 of the present invention show quantum yields for Eu³⁺ luminescence of more than 0.3 under the excitation of visible-light (calculated without deducting the scattering intensity where the light is absorbed). Since the concentration of luminescent rare-earth complexes in the nanoparticles can be quite high, the nanoparticles may have very high luminescent brightness under the excitation of visible-light. Some lanthanide complex-based luminescent nanoparticles of the present invention have better luminescent properties under the excitation of long-wavelength visible-light than same lanthanide complexes dissolved in organic solvent, for example, compared with the Eu(tta)₃dpbt dissolved in toluene, remarkable red-shift is occurred to the excitation peak and the excitation window red edge of the Eu(tta)₃dpbt-based luminescent nanoparticle of the present invention in the visible-light region, the nanoparticles have a higher luminescent brightness under excitation of long-wavelength (Φ×ε). Some luminescent nanoparticles of the present invention also have excellent luminescent properties under two-photon excitation. For example, the aforementioned Eu(tta)₃dpbt, Eu(fod)₃dpbt or Eu(nta)₃bpt based luminescent nanoparticles can emit bright red light under the excitation of near-infrared laser, and the two-photon excitation action cross sections of the rare-earth complexes in the nanoparticles are higher than 30 GM (1 GM = 10⁻⁵⁰ cm⁴ s photo⁻¹ molecule⁻¹).
2. Good stability. In the luminescent nanoparticles provided by the present invention, the lanthanide complex luminescent dyes are dispersed in a hydrophobic macromolecule matrix material since the macromolecular compounds as used are amphiphilic, avoiding their interactions with water molecule and other components in the aqueous solution. The luminescent nanoparticles provided by the present invention have carboxyl groups on their surfaces, making they have very good dispersion stability in aqueous solution and some buffer solutions. The surfaces of the nanoparticles dispersed in water are negatively charged, which contributes to the decrease of non-specific adsorption in the bioassay. The luminescent nanoparticles of the present invention further possess good photostability and thermal stability. For example, the decrease in the luminescent intensity of the nanoparticles as prepared in Examples 1 and 7 is less than 10% after the nanoparticles were illuminated with the excitation light for 1 h.
3. The preparation methods provided by the present invention are simple, and controllable, which solves the problem of the nano-encapsulation of lanthanide complexes with excellent luminescent properties under the excitation of visible-light and near-infrared light, enabling the substantial maintenance or further improvement of the excellent luminescent properties thereof in the nanoparticle.

The carboxyl groups on the surfaces of the luminescent nanoparticles can be used to bond the biomolecules to prepare luminescent nanoprobes with excellent properties. The luminescent nanoparticles provided by the present invention have excellent photoluminescent properties, which makes they be well suitable for synthesizing luminescent biological nanoprobes with high sensitivity, large penetration depth, and low damage to biological samples. A variety of antigens, antibodies, enzymes, DNA, RNA, such as immunoglobulin IgG, α-fetoprotein AFP and the like can be labeled with the luminescent nanoparticles provided by the present invention. The labeled luminescent biological nanoprobes may be used in bioassay, for example high-sensitivity luminescent immunoassay, luminescent bioimaging and biochip and the like.

The lanthanide complexes such as Eu(nta)₃bpt etc. provided by the present invention have the following advantages: such complexes not only have high fluorescence quantum yield (0.52, reference is the n-propanol solution of DCM, 20 °C) but also have an excitation peak of the excitation spectrum at 412 nm with the tail extending up to 460 nm (concentration is 1.0×10⁻⁵ M). However, the complexes such as Eu(tta)₃dpbt, Eu(nta)₃dpbt and the like that have been reported (Y. Wang, et al. Angew. Chem. Int. Ed. 2004, 43, 5010-5013; O. S. Wolfbeis, et al., Anal. Chem. 2006, 78, 5094-5101) have an excitation peak of the excitation spectrum at 403 nm with the tail extending up to about 440 nm (concentration is 1.0× 10⁻⁵ M). As can be seen, the complexes such as Eu(nta)₃bpt etc. provided by the present invention have better long-wavelength sensitized luminescence properties of europium ions. They have important application values in the aspects of bio-sensing, biochemical assay and the like.

## Claims

1. A kind of luminescent nanoparticles comprising a matrix material and a rare-earth complex dispersed in the matrix material;
wherein the matrix material is a macromolecular compound composed of a backbone which is a hydrocarbon chain, a pendant carboxyl group and a pendant hydrophobic group; the rare-earth complex is a rare-earth complex luminescent dye that emits visible-light or near-infrared light under excitation of visible-light and/or near-infrared light and/or ultraviolet light.

2. The luminescent nanoparticles according to claim 1, **characterized in that**: the luminescent nanoparticles consist of the matrix material and the lanthanide complex dispersed in the matrix material.

3. The luminescent nanoparticles according to claim 1 or 2, **characterized in that**: in the matrix material, the hydrophobic group is selected from at least one of alkyl, phenyl, ester group and ether group; the matrix material is selected from at least one of methacrylic acid-methacrylate copolymer, styrene-methacrylic acid copolymer, acrylic acid-acrylate copolymer, styrene-maleic acid copolymer, styrene-maleic anhydride copolymer, styrene-alkyl maleate-maleic acid copolymer, styrene-isobutyl maleate-maleic acid copolymer, alkyl maleate-maleic acid copolymer, copolymer of monobutyl maleate and methyl vinyl ether and styrene-methacrylate-acrylic acid copolymer, preferably at least one of methacrylic acid-methacrylate copolymer, styrene-methacrylic acid copolymer, styrene-maleic acid copolymer, styrene-alkyl maleate-maleic acid copolymer, copolymer of monobutyl maleate and methyl vinyl ether and styrene-isobutyl maleate-maleic acid copolymer, more preferably at least one of methacrylic acid-methyl methacrylate copolymer, styrene-methacrylic acid copolymer, styrene-maleic acid copolymer and styrene-isobutyl maleate-maleic acid copolymer;
the lanthanide complex is selected from at least one of the compounds represented by general structural formula of formula IV, general structural formula of formula V, and general structural formula of formula VI; 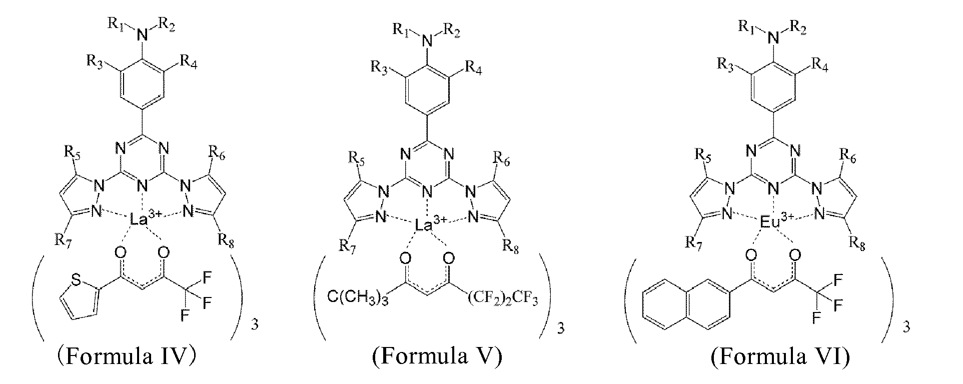 in the general structural formulas of formula IV, formula V and formula VI, La represents europium, ytterbium or neodymium ion; R₁ and R₂ each is selected from any one of alkyl groups with 1-4 carbon atoms, R₃, R₄, R₅, R₆, R₇ and R₈ each is selected from any one of a methyl group and H.

4. The luminescent nanoparticles according to any one of claims 1-3, **characterized in that**: mass ratio of the matrix material to the lanthanide complex is 1∼10,000:1, preferably 3∼1000: 1, more preferably 3∼100: 1; the macromolecular compound has a number average molecular weight of 1,500-150,000, preferably 5,000-100,000, more preferably 10,000-70,000; in the macromolecular compound, the carboxyl group comprises 0.01%∼40% of total mass of the macromolecular compound; the luminescent nanoparticles have a particle size of 3-200 nanometers, preferably 8-120 nm.

5. A method for making the luminescent nanoparticles of any of claims 1-4 comprising the following steps:
1) dissolving the lanthanide complex and the matrix material in an organic solvent that is miscible with water to give an organic solution of the lanthanide complex and the matrix material;
2) mixing the organic solution obtained from step 1) with water;
3) removing the organic solvent in the mixture obtained from step 2), a precipitate is obtained by centrifugation separation from the obtained mixture, and re-dispersed into water or buffer solution, thereby obtaining a sol of the luminescent nanoparticles of any of claims 1-4 dispersed in the water or buffer solution.

6. The method according to claim 5, **characterized in that**:
in the step 1), the lanthanide complex has a concentration of 1×10⁻⁴∼10 g/L, preferably 0.01∼3 g/L; the matrix material has a concentration of 1×10⁻⁴∼100 g/L, preferably 0.01∼10 g/L; the organic solvent that is miscible with water is selected from at least one of methanol, ethanol, acetone, acetonitrile, dimethylformamide and tetrahydrofuran;
in the step 2), the volume ratio of the water to the organic solution obtained from the step 1) is 0.2-1000, preferably 0.5-100;
in the step 3), in the step of removing the organic solvent in the mixture obtained from the step 2), temperature is 4∼100°C, preferably 4∼80°C; the buffer solution is selected from at least one of phosphate buffer solution, Tris-HCl buffer solution and carbonate buffer solution.

7. Use of the luminescent nanoparticles of any of claims 1-4 in preparation of a luminescent biological nanoprobe or a luminescent label for biological imaging.

8. A colloidal solution formed by dispersing the luminescent nanoparticles of any of claims 1-4 in water or buffer solution.

9. The colloidal solution according to claim 8, **characterized in that**: the buffer solution is selected from at least one of phosphate buffer solution, Tris-HCl buffer solution and carbonate buffer solution.

10. Use of the colloidal solution of any of claims 8-9 in preparation of a luminescent biological nanoprobe or a luminescent label for biological imaging.

11. An europium ion complex as represented by the general structural formula of formula I,
in the general structural formula of formula I, R₁ and R₂ each is selected from any one of alkyl groups with 1-4 carbon atoms

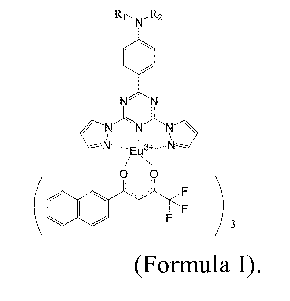

12. The complex according to claim 11, **characterized in that**: in the general structural formula of formula I, R₁ and R₂ each is an ethyl.

13. A method for making the europium ion complex of claim 11 or 12, comprising the following steps:
synthesizing the europium ion complex of claim 11 or 12 by the reaction between the compound as represented by the general structural formula of formula II and the compound as represented by the general structural formula of formula III, 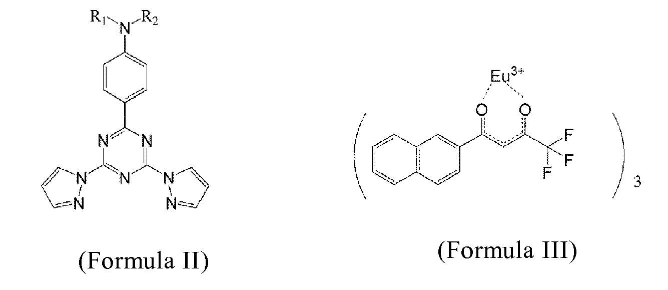 in the general structural formula of formula II, R₁ and R₂ each is selected from any one of the alkyl groups with 1-4 carbon atoms.

14. The method according to claim 13, **characterized in that**: molar ratio of the compound as represented by the general structural formula of formula II and the compound as represented by the general structural formula of formula III is 1:1; in the reaction, the temperature is -10-∼100 °C, preferably 20∼30 °C ; the reaction is performed in an organic solvent, and the organic solvent is selected from at least one of tetrahydrofuran, ethyl ether, benzene, toluene, xylene, chloroform and dichloromethane.
